(19) Europäisches Patentamt
European Patent Office
Office européen des brevets

(11) Publication number: 0 144 220 A2

(12) # EUROPEAN PATENT APPLICATION

(21) Application number: 84308312.2

(22) Date of filing: 29.11.84

(51) Int. Cl.[4]: C 07 C 149/34
C 08 G 75/02

(30) Priority: 30.11.83 GB 8331983

(43) Date of publication of application: 12.06.85 Bulletin 85/24

(84) Designated Contracting States: AT BE DE FR GB IT NL

(71) Applicant: The British Petroleum Company p.l.c.
Britannic House Moor Lane
London EC2Y 9BU(GB)

(72) Inventor: Brown, Peter John Nicholas
The British Petroleum Company p.l.c. Chertsey Road
Sunbury-on-Thames Middlesex, TW16 7LN(GB)

(74) Representative: Harry, John et al,
BP INTERNATIONAL LIMITED Patents Division Chertsey Road
Sunbury-on-Thames Middlesex TW16 7LN(GB)

(54) **Thiofluorophenylenes.**

(57) Novel thiofluorophenylenes and polymers thereof are provided which are prepared by reacting in a polar medium a florinated aromatic compound with a source of sulphide radical.

EP 0 144 220 A2

THIOFLUOROPHENYLENES

The present invention relates to thiofluorophenylenes, polymers thereof and to processes for producing the same.

Production of polyphenylenes by catalytic polymerisation of benzene is well known. The polymerisation is usually effected in the presence of a catalyst comprising a strong Lewis acid and an oxidising agent. It is also known to produce polyphenylenes by the addition of an aryl Grignard reagent or an aryllithium to a substituted cyclic ketone followed by dehydration and aromatisation. However, most of these processes give rise to relatively unstable or intractable products. It is also known from Aroskar E.V. et al in J.C.S. (C) p1569, (1968) that charge transfer complexes may be formed between aromatic hydrocarbon and fluorocarbon rings.

It has now been found that relatively stable compounds can be produced which contain not only phenylene and fluorinated phenylene groups but also sulphur groups in a mixed distribution.

Accordingly, the present invention is a thiofluorophenylene compound of the formula

$$-(Z_b-S)_n- \qquad (I)$$

in which Z is any combination of phenylene, polyphenylene, and fluorosubstituted derivatives thereof such that Z contains at least one phenylene group with at least two fluorine substituents,

b is at least 1, and

n is at least 2.

According to a second aspect the present invention is a poly-(thiofluorophenylene) compound of the formula

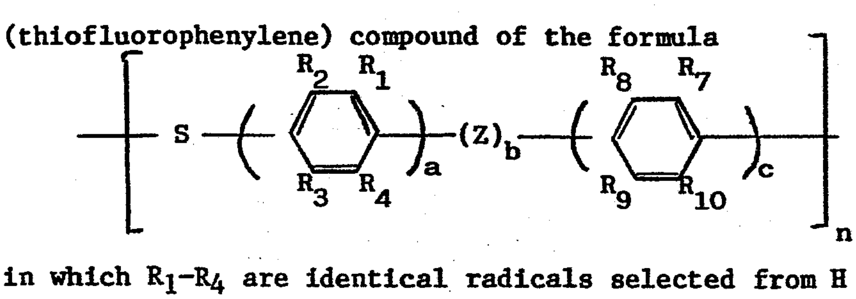

(II)

in which $R_1$-$R_4$ are identical radicals selected from H and F, $R_7$-$R_{10}$ are identical radicals selected from H and F, Z, n and b are as defined in formula (I) above, a is an integer from 1 to 50, and c has a value from 0 to 10 such that a+b+c is at least 2.

By 'any combination of phenylene, polyphenylene and fluorosubstituted derivatives thereof' is meant phenylene groups, biphenyls and polyphenyls which carry at least two fluorine substituents which may be in the ortho-, meta- or in the para-position, and in the case of bi- and polyphenyls the phenylene groups may be linked in the ortho-, meta- or para-position with respect to the sulphur atom.

Specific examples of the thiofluorophenylenes according to the present invention include but are not limited to

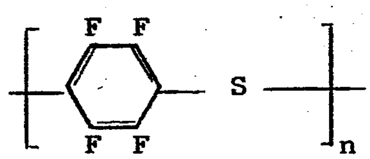

(III)

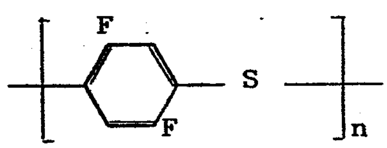

(IV)

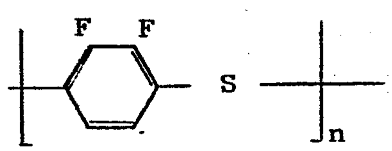

(V)

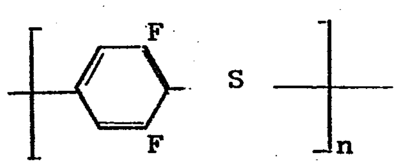

(VI)

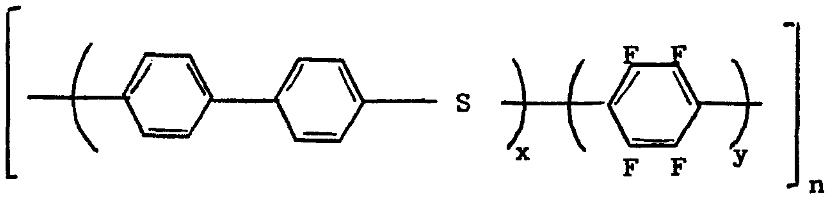

(VII)

where each of x and y are at least 1, and

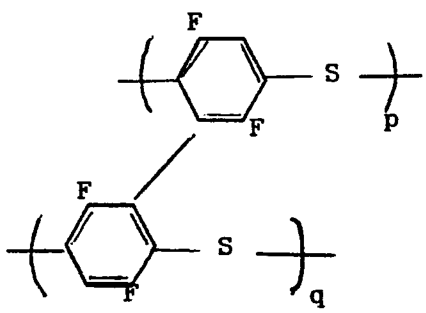

(VIII)

wherein each of p and q is at least 1.

The thiofluorophenylene compounds of the present invention may be produced in various ways depending upon the reactants used and the products desired.

In one embodiment of the invention, thiofluorophenylene compounds may be produced by reacting a fluorinated aromatic hydrocarbon with a source of sulphide radicals such as e.g. hydrogen sulphide, sodium sulphide, sodium hydrogen sulphide or other metal sulphides or hydrogen sulphides and/or by reacting a fluorinated aromatic hydrocarbon containing sulphide links with a lithiated derivative of a fluorinated or non-fluorinated aromatic hydrocarbon.

Examples of suitable fluorinated aromatic hydrocarbon and/or lithiated derivatives thereof that may be reacted in this manner include compounds of the formula

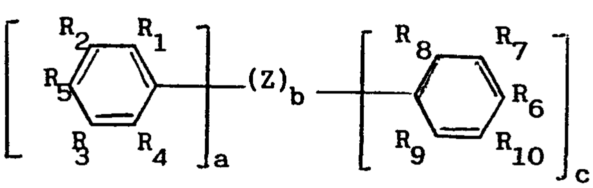

(IX)

wherein each of $R_1$-$R_4$ and $R_7$-$R_{10}$ represent H or F atoms, each of $R_5$ and $R_6$ are identical atoms selected from a halogen and lithium, and each of Z, a, b and c are as defined in formula (II) above.

Illustrative examples of compounds that may be used as starting materials are those represented by formula (IX) above and include the following in which X represents a halogen atom or a lithium atom.

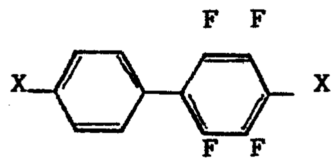

(X)

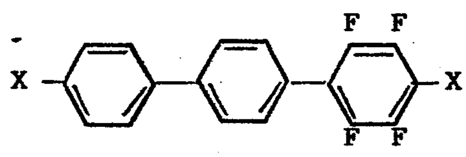

(XI)

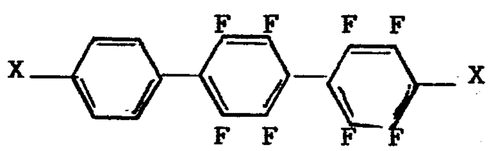

(XII)

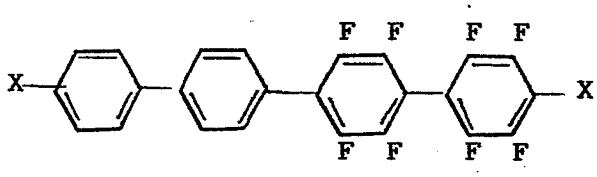

(XIII)

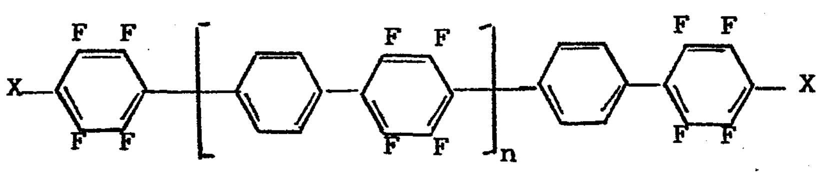

(XIV)

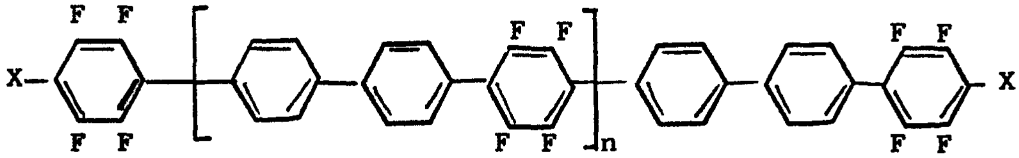
(XV)

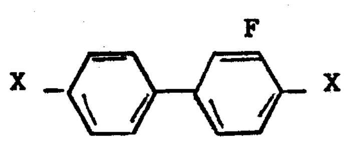
(XVI)

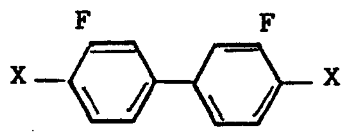
(XVII)

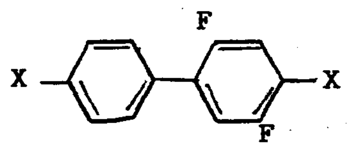
(XVIII)

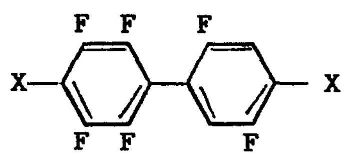
(XIX)

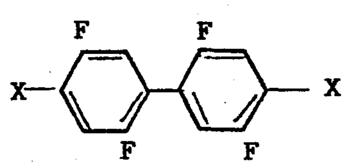
(XX)

Any of the compounds falling within the formula (IX) e.g. those represented by formulae (X)-(XX) may be reacted either with hydrogen sulphide, sodium sulphide, sodium hydrogen sulphide or other metal sulphides or hydrogen sulphides or with a fluorinated aromatic

hydrocarbon containing sulphide links to produce the thiofluoro phenylene compounds of the present invention.

The reaction may be carried out suitably in a homogeneous or heterogeneous phase and may be in the liquid-liquid, liquid-vapour, liquid-solid or solid-vapour phases. The reaction is preferably carried out in a liquid phase, more preferably in a polar liquid medium. Examples of suitable liquid reaction media that may be used include N,N-dimethyl formamide, N-methyl pyrrolidone and other dipolar aprotic solvents. For lithiated derivatives the solvent used is preferably inert under the reaction conditions and may be selected from tetrahydrofuran, diethylether and the like.

If it is desired to produce a film of the thiofluorophenylene in its polymer form, it is preferable to have at least one of the reactants in the vapour phase. For instance, it is possible to form a film of the thiofluorophenylene polymer by passing hydrogen sulphide over a preformed film of the fluorinated phenylene or polyphenylene.

The reaction is suitably carried out at a temperature from -80°C to +200°C depending upon the nature of the reactants and products desired. Similarly, the reaction may be carried out at ambient or elevated pressures.

The relative concentrations of the fluorinated aromatic hydrocarbon and/or its lithiated derivative, and the sulphide reactants will also depend upon the products desired. For most reactions it is preferable to have the two reactants in equimolar proportions although a molar excess of either may also be used.

The reaction products either separate from the liquid reaction medium or may be separated or recovered by pouring the reaction mixture, upon completion of reaction, into water. Water enables reactants such as sodium sulphide or the reaction medium such as N,N-dimethylformamide to be removed.

The process of the present invention may be used to produce thiofluorophenylenes falling within the formula (I) above. Specifically these may be represented by:

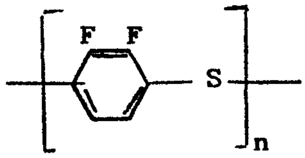

(XXI)

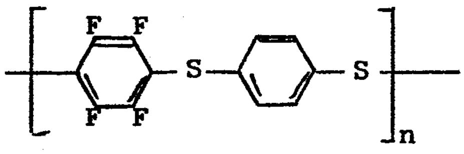

(XXII)

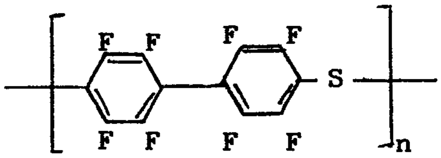

(XXIII)

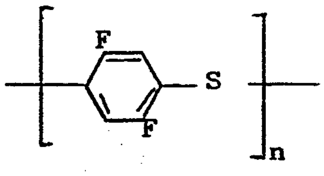

(XXIV)

and by compounds which are crosslinked and have a molecular formula $(C_{12}H_2F_4S_2)n/2$ and may be represented by:

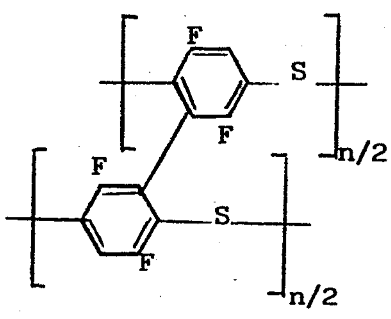

(XXV)

The thiofluoro(poly)phenylene products of the present invention have inherent electrical properties although in some cases these may

not be noticeable to any significant extent. The polymers of the present invention may however be doped using conventional dopants such as the halogens, fluorides of arsenic, halides of molybdenum, protonic acids etc to enhance their conducting properties.

The present invention is further illustrated with reference to the following examples. In all the Examples the alphabetical designations used in the spectroscopic data have the following significance: w = weak, m = medium, s = strong, vs = very strong and sh = shoulder.

Example 1

Sodium sulphide $9H_2O$ (1.20g) was added to N,N-dimethylformamide (20 ml) and was well stirred at room temperature. Decafluorobiphenyl (1.67g) was then added and the mixture was stirred at room temperature for 72 hours.

Water (100 ml) was then added and well mixed. The resulting mixture was filtered to give a solid which was shaken with a further amount of water (100 ml) and then filtered and dried to give a solid which in turn was shaken with diethyl ether (50 ml). This mixture was then filtered and dried to give the final solid product (1.12g); melting point greater than 300°C.

Mass spectrometry of this product showed the presence of a range of molecules corresponding to the structures having increasing molecular weight up to that of

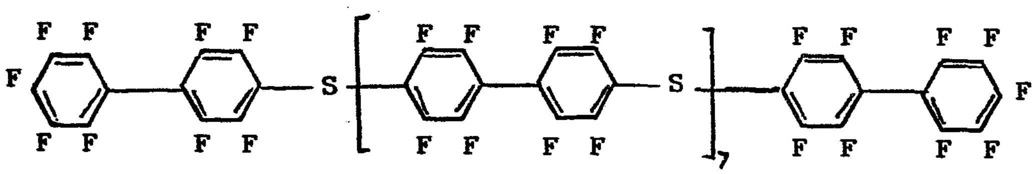

with peaks at M/z 662, M/z 990, M/z 1318, M/z 1646, M/z 1974, M/z 2302, m/z 2630 and M/z 2958, the spectrum being limited at this point by the lack of volatility of the sample. Infra-red spectroscopy showed major peaks at 1645 (m), 1530 (m, sh, sharp), 1505 (w, sh), 1470 (vs), 1265 (m, sharp), 1245 (m–s), 1130 (m), 1000 (s), 960 (s), 895 (m) and 721 (s, sharp) $cm^{-1}$. Raman bands were observed at the following wave number values; 153 (w), 408 (m),

0144220

444 (w), 518 (m), 622 (w), 1066 (m), 1131 (m), 1301 (w) and 1649 (vs) $cm^{-1}$.

Example 2

Sodium sulphide $9H_2O$ (2.40g) was added to N,N-dimethylformamide (20 ml) and was well stirred at room temperature. 1,2,4,5- Tetrafluorobenzene (1.50g) was then added and the mixture was stirred at room temperature for 6 hours. The mixture was then heated to 110°C and kept at 110°C with good stirring for 6 hours before being boiled under reflux with good stirring for a further 7 hours.

After cooling, the mixture was poured into water (150 ml) and well mixed before being filtered to give a solid (0.60g). This solid was crushed and dried and then well mixed with diethyl ether (25 ml). Filtration gave a solid which was washed with further diethyl ether (25 ml) and dried to give the final solid product (0.50g); melting point greater than 300°C.

Mass spectrometry of this product showed the presence of a range of molecules corresponding to the structures having increasing molecular weight up to that of

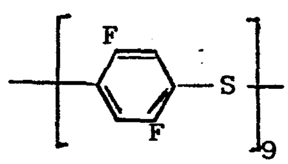

with groups of peaks with a repeating pattern 144 apart, the spectrum being limited at the level of nine units by the lack of volatility of the sample. Infra-red spectroscopy showed major peaks at 1611 (m), 1595 (m - w), 1500 (w), 1464 (vs), 1425 (w), 1370 (s) 1342 (w), broad band with sub-maxima at 1260 (w) and 1245 (w) 1174 (s), 1082 (m-w), 1055 (w), 1035 (w), 965 (m-w), 875 (s) and 785 (s) $cm^{-1}$. Raman bands were observed at the following wave number values; 249 (m), 354 (m), 398 (w), 463 (m), 492 (w), 690 (m, sh), 704 (m-s), 860 (w), 969 (s), 1122 (m-w), 1140 (m-w), 1214 (w), 1263 (s), 1544 (w) and 1596 (s) $cm^{-1}$.

Claims:

1. A thiofluorophenylene compound of the formula

$$-(Z_b-S)_n- \qquad (I)$$

in which Z is any combination of phenylene, polyphenylene, and fluorosubstituted derivatives thereof such that Z contains at least one phenylene group with at least two fluorine substituents,

b is at least 1, and

n is at least 2.

2. The compound of Claim 1 wherein Z is only a phenylene group with a least two fluorine substitutents.

3. A poly(thiofluorophenylene) compound of the formula

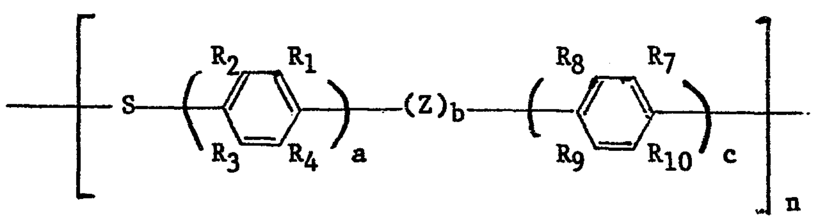

(II)

in which $R_1$-$R_4$ are identical radicals selected from H and F,

$R_7$-$R_{10}$ are identical radicals selected from H and F,

Z, n and b are as defined in formula (I) above,

a is an integer from 1 to 50, and

c has a value from 0 to 10 such that a+b+c is at least 2.

4. The compound of Claim 3 wherein

$R_1$-$R_4$ are H,

Z is one or more phenyl groups bonded to a sulphur atom,

$R_7$ $R_{10}$ are F, and

a and c are as previously defined.

0144220

5. A method of preparing the compounds of Claim 1 and Claim 2, the method comprising reacting in a polar liquid medium a fluorinated aromatic compound with a source of sulphide radicals at a temperature from -80°C to +200.

6. The method of Claim 5 wherein the liquid medium is selected from N,N-dimethyl formamide and N-methyl pyrrolidone.

7. The method of Claim 6 wherein the fluorinated aromatic hydrocarbon and the source of sulphide radicals are reacted in equimolar proportions.